# EUROPEAN PATENT APPLICATION

(11) **EP 0 543 023 A1**
(43) Date of publication of application: **26.05.1993**
(21) Application number: 92911402.3
(22) Date of filing: 04.06.1992
(51) Int. Cl.: C12N 1/16, C12P 23/00

(54) **PROCESS FOR PRODUCING ASTAXANTHIN BY FERMENTATION**

(30) Priority: 06.06.1991 JP 134750/91
(71) Applicant: KYOWA HAKKO KOGYO KABUSHIKI KAISHA, Chiyoda-ku, Tokyo 100 (JP)
(72) Inventor: KUGA, Tetsuro, Hofu-shi Yamaguchi 747 (JP); INOUE, Makoto, Hofu-shi Yamaguchi 747 (JP); IMURA, Toshiaki, Hofu-shi Yamaguchi 747 (JP); AOYAMA, Yoshihide, Hofu-shi Yamaguchi 747 (JP); KURATSU, Yoshiyuki, Hofu-shi Yamaguchi 747 (JP)
(74) Representative: VOSSIUS & PARTNER
(86) International application number: JP9200722
(87) International publication number: WO9221764

(57) **Abstract**

A process for producing astaxanthin industrially efficiently at a low cost by culturing a microorganism which belongs to the genus *Pfaffia*, is resistant to at least one of citronellol, primaquine and hydroxydiphenyl and can produce astaxanthin in a medium.

## Description

### Technical Field

The present invention relates to a process for producing astaxanthin by fermentation. Astaxanthin is a naturally occurring carotenoid and is used to improve coloration of fishes in commercial aquaculture.

### Prior Art

Heretofore, various methods for producing astaxanthin using a microorganism of the genus Phaffia are known, among these, a method of using a microorganism belonging to the genus Phaffia and having sensitivity to antimycin-A [Applied and Environmental Microbiology, 55, 116, (1989)]; a method of using a micrcorganism belonging to the genus Phaffia and having resistance to b-ionon [ibid, 56, 2944, (1990)]; a method of using Phaffia rhodozyma ATCC24202 (Japanese Published Examined Patent Application No. 61907/88); a method of using a mutant strain of a microorganism of the genus Phaffia which is obtained by a mutagenesis with ethyl methanesulfonate, UV irradiation, or N-methyl-N'-nitro-N-nitrosoguanidine [Japanese Published Unexamined Patent Application No. 504101/90 (WO 88/08025)], a method of using a microorganism belonging to the genus Phaffia and having resistance to geraniol [Japanese Published Unexamined Patent Application No. 206880/91 (EP 427405)]; and a method of using a microorganism belonging to the genus Phaffia and having resistance to antibiotics (antimycin, tunicamycin, nystatin), cytochrome-b inhibitors (antimycin, 2-n-heptyl-4-hydroxyquinoline-N-oxide), or terpenoid synthesis pathway inhibitors (mevalonic lactone) [Japanese Published Unexamined Patent Application No. 501053/92 (WO 90/01552)].

### Disclosure of the Invention

According to the present invention, astaxanthin can be industrially produced in a cheap and efficient manner by culturing in a medium a microorganism which belongs to the genus Phaffia and has both resistance to at least one of citronellol, primaquine and hydroxydiphenyl and the ability to produce astaxanthin, and recovering astaxanthin therefrom.

Also, the present invention provides a culture, cells or processed cells of a microorganism belonging to the genus Phaffia and having both resistance to at least one of citronellol, primaquine and hydroxydiphenyl and the ability to produce astaxanthin.

The processed cells in the present invention include a mechanically ground product, an ultrasonically treated product, a solvent treated product, an enzymatically treated product, a surfactant treated product and, a dried product of cells.

In the specification, the expression "resistance to citronellol, primaquine or hydroxydiphenyl" means the ability to grow in the presence of 220 µg/ml of citronellol, 3.9 g/ℓ of primaquine or 33 µg/ml of hydroxydiphenyl.

As the microorganism used in the present invention, any microorganism can be used so long as it belongs to the genus Phaffia and it has both resistance to at least one of citronellol, primaquine and hydroxydiphenyl and the ability to produce astaxanthin. A strain of the microorganism is obtained by subjecting astaxanthin-producing microorganism of the genus Phaffia to a conventional method of mutagenesis such as ultraviolet irradiation and treatment with a mutagenic agent (N-methyl-N'-nitro-N-nitrosoguanidine, etc.), and then by selecting, from the resultant mutants, those which can grow on a medium containing citronellol, primaquine or hydroxydiphenyl. Examples of such microorganisms include those belonging to the genus Phaffia rhodozyma, for example, Phaffia rhodozyma H-8264 (hereafter referred to as H-8264 strain), Phaffia rhodozyma H-8434 (hereafter referred to as H-8434 strain) and Phaffia rhodozyma H-8435 (hereafter referred to as H-8435 strain), etc.

A specific process for obtaining the above mentioned mutant strain is shown below.

Phaffia rhodozyma ATCC24202 (hereafter referred to as ATCC24202 strain) was used as the parent strain. After treating the strain with 0.02 ml/ml ethyl methanesulfonate at 22°C for 60 minutes, it was spread on a minimal agar plate culture medium (20 g/ℓ glucose, 7 g/ℓ yeast nitrogen base of Difco Laboratories, 20 g/ℓ agar) containing citronellol at a concentration (220 µg/ml) at which the growth of the parent strain was inhibited. After culturing at 22°C for 7-10 days, those strains were selected, from the growing mutant strains, which exhibited superior ability to produce astaxanthin to the parent strain in liquid culture test done in a 250 ml-Erlenmeyer flask. Out of these, H-8264 strain was selected as a particularly superior strain.

Also, out of the strains obtained in the same manner mentioned above except for using primaquine (3.9 g/ℓ) or hydroxydiphenyl (33 µg/ml) instead of citronellol, H-8434 strain or H-8435 strain was obtained as a strain with particularly superior ability to produce astaxanthin.

The resistance of these mutant strains was examined in the following manner.

The mutant strain (H-8264 strain, H-8434 strain or H-8435 strain) and the parent strain (ATCC24202 strain) were spread on a minimal agar plate culture medium containing additives at the concentrations shown in Tables 1-3, and cultured at 22°C. The results are shown in Tables 1-3.

**Table 1**

| Citronellol (µg/ml) | Strain | |
|---|---|---|
| | ATCC24202 | H-8264 |
| Not added | + | + |
| 200 | + | + |
| 220 | - | + |
| Note: +: growth -: no growth | | |

**Table 2**

| Primaquine (g/ℓ) | Strain | |
|---|---|---|
| | ATCC24202 | H-8434 |
| Not added | + | + |
| 3.9 | - | + |
| 6.5 | - | + |
| Note: +: growth -: no growth | | |

**Table 3**

| Hydroxydiphenyl (µg/ml) | Strain | |
|---|---|---|
| | ATCC24202 | H-8435 |
| Not added | + | + |
| 30 | ± | + |
| 33 | - | + |
| Note: +: growth ±: slight growth -: no growth | | |

Each of the three strains mentioned above, namely, H-8264 strain, H-8434 strain and H-8435 strain was deposited with the Fermentation Research Institute, Agency of Industrial Science and Technology, Japan, under the Budapest Treaty, on May 18, 1991, March 13, 1992 and March 13, 1992 with the accession numbers FERM BP-3404, 3800 and 3801.

As the medium used in the present invention, any of a synthetic medium and a natural medium may be used so long as it suitably contains carbon sources, nitrogen sources, inorganic salts, or growth factors, etc.

As the carbon source, glucose, fructose, sucrose, molasses, etc. may be used. As the nitrogen source, ammonia, ammonium sulfate, peptone, yeast extract, corn steep liquor, etc. may be used. As the inorganic salt, potassium dihydrogenphosphate, dipotassium hydrogen-phosphate, magnesium phosphate, magnesium sulfate, ferrous sulfate, manganese sulfate, calcium chloride, etc. may be used. As the growth factors, vitamins, amino acids, nucleic acid related substances, etc. may be used.

Culturing is carried out by batch culture or continuous culture at a temperature of 15 to 35°C, preferably 20 to 25°C, and at pH 3 to 8, preferably 4 to 6, and generally completed in 2 to 7 days. The pH of the medium is adjusted with calcium carbonate, an inorganic or organic acid, an alkali solution, ammonia, a pH buffer (e.g., potassium hydrogen phthalate), etc.

After the completion of the culturing, in order to isolate the cells (containing astaxanthin) or astaxanthin itself from the culture, the cells are harvested, dried and ground; or the cells are harvested from the culture and ruptured, after which astaxanthin is extracted from the ruptured cells with solvent, and the extract is subjected to silica gel chromatography.

Astaxanthin, and cultures, cells and processed cells, etc. of the above microorganisms obtained according to the present invention are used as feed for fish and crustacea.

### Best Mode for Carrying Out the Invention

Examples are shown below.

### Example 1

Each of H-8264, H-8434 and H-8435 strains was inoculated on 8 ml of a seed medium having the following composition in a test tube, and cultured at 22°C for 3 days.

Composition of seed medium: 10 g/ℓ glucose, 3 g/ℓ yeast extract, 5 g/ℓ peptone, 3 g/ℓ meat extract (pH 5.0, adjusted with KOH, autoclaved at 120°C for 20 minutes).

Three ml of each of the 3 seed culture broth thus obtained was inoculated into 30 ml of a production medium having the composition shown below in a 250 ml-Erlenmeyer flask, and cultured at 22°C for 4 days.

Composition of production medium: 30 g/ℓ glucose, 2 g/ℓ ammonium sulfate, 2 g/ℓ yeast extract, 1 g/ℓ KH₂PO₄, 0.5 g/ℓ MgSO₄·7H₂O, 0.1 g/ℓ CaCl₂·2H₂O, 20.4 g/ℓ potassium hydrogen phthalate (pH 5.0, adjusted with KOH, autoclaved at 120°C for 20 minutes).

The amounts of astaxanthin produced in the culture of H-8264 strain, H-8434 strain and H-8435 strain were 5.8 mg/ℓ, 5.5 mg/ℓ and 4.7 mg/ℓ, respectively.

On the other hand, the parent strain ATCC24202, which was used for comparison, was cultured in the same manner described above, and the amount of astaxanthin produced in a culture was 3.3 mg/ℓ.

### Example 2

H-8264 strain was inoculated into 300 ml of the seed medium described in Example 1 in a 2 ℓ-Erlenmeyer flask, and cultured at 22°C for 3 days. Thereafter 300 ml of the obtained seed culture was inoculated into 3 ℓ of a production medium, in a 5 ℓ-jar fermentor, having the same composition described in Example 1 except that potassium hydrogen phthalate was not contained, and cultured at 22°C for 48 hours (agitation 600 rpm, aeration 3 ℓ/min). The pH during the culturing was adjusted to 5 with aqueous ammonia. As a result, the amount of astaxanthin produced in the culture was 17.1 mg/ℓ.

The cells were harvested from the culture and ruptured with a homogenizer. Astaxanthin was extracted from the ruptured cells with acetone, and the extract was concentrated under reduced pressure. The concentrate was subjected to silica gel chromatography (eluent; acetone:petroleum ether (1:19 v/v) Æ acetone:petroleum ether {1:4 v/v} concentration gradient) to obtain 32 mg of astaxanthin.

### Example 3

H-8434 strain was inoculated into 300 ml of the seed medium described in Example 1 charged in a 2 ℓ-Erlenmeyer flask, and cultured at 22°C for 3 days. Eighty ml of the resulting seed culture was inoculated into 720 ml of a production medium, in a 2 ℓ-jar fermentor, having the same composition described in Example 1 except that potassium hydrogen phthalate was not contained, and cultured at 22°C for 48 hours (agitation 900 rpm, aeration 0.8 ℓ/min). The pH during the culturing was adjusted to 5 with aqueous ammonia. The amount of astaxanthin produced in the culture was 16.3 mg/ℓ.

After being harvested from the culture, the cells were dried with a spray type dryer to yield 37 g of dried cells containing 14 mg of astaxanthin.

## Claims

1. A process for producing astaxanthin by fermentation which comprises culturing in a medium a microorganism belonging to the genus Phaffia and having both resistance to at least one of citronellol, primaquine and hydroxydiphenyl and the ability to produce astaxanthin, accumulating astaxanthin in the culture broth, and recovering astaxanthin therefrom.

2. A culture broth, cells or processed cells of a microorganism belonging to the genus Phaffia and having both resistance to at least one of citronellol, primaquine or hydroxydiphenyl and the ability to produce astaxanthin.
